# EUROPÄISCHE PATENTSCHRIFT

(11) **EP 0 960 619 B1**
(45) Veröffentlichungstag und Bekanntmachung des Hinweises auf die Patenterteilung: **10.03.2004**
(21) Anmeldenummer: 99109927.6
(22) Anmeldetag: 20.05.1999
(51) Int. Cl.: A61K 31/215, A61K 47/38

(54) **Retardiertes Schmerzmittel enthaltend Tilidin**
Sustained release analgesic compositions comprising tilidine
Formulations analgésiques de tilidine a liberation prolongée

(30) Priorität: 15.12.1998 DE 19857766; 28.05.1998 DE 19823778
(43) Veröffentlichungstag der Anmeldung: 01.12.1999
(73) Patentinhaber: Krewel Meuselbach GmbH, 53783 Eitorf (DE)
(72) Erfinder: Schierstedt, Detlef Dr., c/oKrewel Meuselbach GmbH, 53783 Eitorf (DE)
(74) Vertreter: Jönsson, Hans-Peter, Dr.Dipl.-Chem.

(56) Entgegenhaltungen:
- WO-A-94/10129
- US-A- 4 070 494
- VETTER C.: "[ Tilidine /naloxone: New formula of an old preparation]." PHARMAZEUTISCHE ZEITUNG, (1997) 142/47 (51). , XP002116565
- BUCK G.: "[ Sustained release tilidine: New options for pain therapy]. SCHMERZTHERAPIE." ZEITSCHRIFT FUR ALLGEMEINMEDIZIN, (5 APR 1999) 75/7 (350-352). , XP002116566
- "ROTE LISTE 1999" 1999 , EDITION CANTOR VERLAG , AULENDORF, DEUTSCHLAND XP002116567 Siehe Eintrag Nr. 05 075

## Beschreibung

Gegenstand der Erfindung sind feste, peroral applizierbare, Retardiermittel aufweisende Schmerzmittel enthaltend Tilidin.

Tilidin, [(+/-)-Ethyl-(trans-2-dimethylamino-phenyl-3-cyclohexen-trans-1-carboxylat)] ist ein entfernt mit Morphin verwandtes Opioidanalgetikum, das enteral rasch resorbiert wird und sich dementsprechend in der Schmerztherapie hervorragend eignet.

In der EP 0 665 830 B1 wird beschrieben, daß vornehmlich Salze des basischen Wirkstoffs in Frage kommen, da die Base als solche keine ausreichende Stabilität über einen längeren Zeitraum aufweist. Es wird weiterhin festgehalten, daß alle Anstrengungen, brauchbare feste Arzneimittelformen des Wirkstoffs Tilidin bereitzustellen, an Stabilitätsproblemen gescheitert sind. Es wird angenommen, daß nur über eine feste Formulierung eine steuerbare Retardierung des Tilidins realisierbar sein dürfte. So wird in der genannten EP 0 665 830 B1 festgehalten, daß Tilidindihydrogenorthophosphat eine hervorragende Stabilität aufweist und sich als bisher einziges Salz für die Herstellung von festen Arzneimittelnformen eignet, weil es in fester Form, d. h. in Verbindung mit festen Hilfsstoffen praktisch keine Zersetzung erleidet. Auch soll das Phosphatsalz überraschend pharmazeutischtechnologische Eigenschaften haben, die die Eigenschaften vergleichbarer Tilidinsalze, wie zum Beispiel das Tilidinhydrogensulfat oder das Tilidinhydrogenfumarat übertreffen.

Tilidin wird im Handel üblicherweise zusammen mit einem Morphinantagonisten, beispielsweise Naloxon-Hydrochlorid eingesetzt.

Es bestand daher die Aufgabe, feste, peroral applizierbare, Retardiermittel aufweisende Schmerzmittel zur Verfügung zu stellen, bei denen die in-vitro Freisetzungsrate des Tilidins und des Morphinantagonisten im wesentlichen gleich sind.

Die vorgenannte Aufgabe wurde gelöst durch feste peroral applizierbare, Schmerzmittel, enthaltend Tilidin als Salz eines nicht toxischen Komplexbildners für 2- oder 3-wertige Metallkationen, ausgewählt aus organischen Säuren und einem Morphinantagonisten in einer Cellulosederivatmatrix als Retardiermittel.

Eine weitere Ausführungsform der vorliegenden Erfindung betrifft feste peroral applizierbare Schmerzmittel enthaltend ein pharmakologisch unbedenkliches Tilidinsalz, **zusätzlich** einen Komplexbildner für 2- oder 3-wertige Metallkationen und einen Morphinantagonisten in einer Cellulosederivatmatrix als Retardiermittel.

Erfindungsgemäß wurde somit gefunden, daß ausgewählte Tilidinsalze von Komplexbildnem für 2- oder 3-wertige Metallkationen nicht nur eine hohe Stabilität im Vergleich zu festen, Tilidin enthaltenden Schmerzmitteln des Standes der Technik aufweisen, sondern daß darüber hinaus auch bei Einsatz der genannten Komplexbildnem in der genannten Matrix die in-vitro Freisetzungsraten des Tilidins und des Morphinantagonisten im wesentlichen gleich sind.

Während es bisher üblich war, feste perorale Schmerzmittel zu retardieren, die nur einen Wirkstoff enthalten, werden erfindungsgemäß zwei Wirkstoffe mit unterschiedlichen chemischen Strukturen und physikalischen Eigenschaften in einer Matrix miteinander kombiniert. Diese Kombination ergibt üblicherweise zwei verschiedene Freisetzungsprofile der beiden Wirkstoffe. Die gleiche Matrix ist aber bei einem festen peroralen Schmerzmittel hoch erwünscht, da beispielsweise die Tablette teilbar und somit eine individuelle Dosierung ermöglicht werden kann. Aus medizinischer Sicht ist für eine retardierte Tilidin/Naloxon-Zubereitung eine vergleichbare, d. h. im wesentlichen gleiche Freisetzung beider Wirkstoffe und eine Teilbarkeit der Darreichungsform hoch erwünscht. Überraschenderweise wurde gefunden, daß in einer Cellulosederivatmatrix Tilidin und Naloxon-Hydrochlorid mit im wesentlichen gleichen Freisetzungsraten freigesetzt werden.

Erfindungsgemäß werden somit insbesondere Tilidinsalze von Komplexbildnem 2-oder 3-wertiger Metallkationen eingesetzt. Besonders bevorzugt in diesem Sinne sind organische Säuren, insbesondere Dicarbonsäuren und/oder Tricarbonsäuren, gegebenenfalls hydroxysubstituiert, beispielsweise Citronensäure und/oder Weinsäure bedingt durch die fehlende Toxizität. Wenn die Komplexbildner neben den Tilidinsalzen als weiterer Bestandteil des Schmerzmittels enthalten sind, so können diese selbstverständlich auch in Form der Alkali- und/oder Erdalkalimetallsalze enthalten sein.

Die genannten Komplexbildner wirken offensichtlich stabilisierend auf das Gesamtsystem und sind in Kombination mit geeigneten Retardiermitteln geeignet, die in-vitro Freisetzungsrate des Tilidins und des Morphinantagonisten im wesentlichen gleichzusetzen. Somit ist es im Sinne der vorliegenden Erfindung möglich, Tilidinhydrochlorid-Semihydrat, Tilidinhydrogenfumarat und Tildinhydrogensulfat jeweils mit Naloxonhydrochlorid-Dihydrat und üblicherweise pharmazeutisch eingesetzten Hilfsstoffen in eine stabile Zubereitung einzubringen. Auch ohne den Zusatz von Komplexbildnern ist in der Cellulosederivat-Matrix eine stabile pharmazeutische Zusammensetzung auf der Basis von Tilidinhydrochloridsemihydrat erhältlich. Gleiches gilt selbstverständlich auch für das in der EP 0 665 830 B1 genannte Tilidinhydrogenorthophospat.

Setzt man den vorgenannten Tilidinsalzen von Komplexbildnern in einer Menge von 0,2 bis 30 Gew.-%, insbesondere 2 bis 6 Gew.-%, bezogen auf die Masse der Gesamtzubereitung zu, so ist auch bei Lagerversuchen bei erhöhter Temperatur keine Instabilität und insbesondere kein aggressives Korrosionsverhalten erkennbar.

Der neben Tilidin eingesetzte Morphinantagonist ist im Sinne der vorliegenden Erfindung besonders bevorzugt ausgewählt aus Naloxonhydrochlorid-Semihydrat.

Zur Herstellung retardierter fester Arzneimittelformen des Tilidins kommen prinzipiell alle üblichen Retardierverfahren in Frage, die die Stabilität des Wirkstoffs aufgrund ihrer Zusammensetzung nicht negativ beeinflussen. So sind in der EP 0 043 254 B durch ein Schmelzverfahren retardierte Tabletten beschrieben. Als Retardiermittel eignen sich aber auch prinzipiell schwerlösliche Stoffe, wie zum Beispiel Lipide oder lipoide Substanzen, wie Stearinsäure und insbesondere hydriertes Rizinusöl (Cutina®HR) (DE 1 617 657 A, US-4,123,753 A) oder hydrophile Polymere, die als Quellstoffe die Freigabe des Wirkstoffs retardieren (J. Pharm. Sci. S. 974 (1966)). Zur gezielten Steuerung der Freisetzung von Tilidin wird in der EP 0 068 446 B ein Verfahren beschrieben, bei dem die Freigabe der Geschwindigkeit des Wirkstoffs aus einer mittels schwerlöslicher Substanzen retardierten Wirkstoffzubereitung durch die Viskosität eines zugegebenen hydrophilen Polymeren, wie zum Beispiel Methylcellulose oder Carboxymethylcellulose, eingestellt wird, ausgehend von der Erkenntnis, daß die Freigabegeschwindigkeit mit steigender Viskosität zunimmt.

Im Sinne der vorliegenden Erfindung sind daher die Retardiermittel ausgewählt aus hydrophilen oder hydrophoben Polymeren, d. h. Cellulosederivaten, insbesondere Alkylcellulosen oder Hydroxyalkylcellulosen mit 1 bis 6 C-Atomen im Alkyl- oder Hydroxyalkylrest. Besonders bevorzugt im Sinne der vorliegenden Erfindung sind daher die Alkylcellulosen ausgewählt aus Methylcellulose oder Ethylcellulose. In gleicher Weise sind die Hydroxyalkylcellulosen besonders bevorzugt ausgewählt aus Hydroxyethylcellulose oder Hydroxypropylcellulose.

Wenn im Sinne der vorliegenden Erfindung definiert wird, daß die in-vitro Freisetzungsraten des Tilidins und des Morphinantagonisten im wesentlichen gleich sind, so bedeutet dies, daß die Freisetzungsraten wie folgt bestimmt worden sind:

Die in-vitro Freisetzung von Tilidinsalz und Morphinantagonist aus den Arzneimittelzubereitungen wurde nach DAB10 (Deutsches Arzneibuch, 10. Auflage) in einer Blattrührerapparatur bestimmt. Die Temperatur des Lösungsmediums betrug 37 °C und die Umdrehungsgeschwindigkeit des Rührers 100 U/min. Zu Beginn der Untersuchung wurde jede Tablette in 900 ml künstlichen Magensaft gegeben. Die zu dem vorbestimmten Zeitpunkt im Lösungsmedium befindliche freigesetzte kombinierte Wirkstoffmenge wurde mittels HPLC bestimmt.

Besonders bevorzugt im Sinne der vorliegenden Erfindung wird die in-vitro Freisetzungsrate demgemäß eingestellt, daß die Freisetzungsgeschwindigkeit des Tilidins nach
1 Stunde 15 bis 30 Gew.-%,
2 Stunden 25 bis 40 Gew.-%,
4 Stunden 50 bis 70 Gew.-%
6 Stunden 60 bis 80 Gew.-% und
8 Stunden 70 bis 90 Gew.-% beträgt.

Darüber hinaus ist es im Sinne der vorliegenden Erfindung besonders bevorzugt, daß sich die Freisetzungraten von Tilidin und Morphinantagonist nach
1 Stunde um höchstens 25 %, bezogen auf Tilidin,
2 Stunden um höchstens 20 %, bezogen auf Tilidin,
4 Stunden um höchstens 15 %, bezogen auf Tilidin,
6 Stunden um höchstens 12 %, bezogen auf Tilidin und
8 Stunden um höchstens 10 %, bezogen auf Tilidin unterscheiden.

Neben der Retardiermatrix können die erfindungsgemäßen Schmerzmittel auch übliche im Stand der Technik bekannte pharmazeutische Hilfsstoffe enthalten, wie beispielsweise Fette, Fettalkohole, Fettsäuren, Acrylate, Alkylacrylate, Tenside, mikrokristalline Cellulose, Stärken, Milchzucker, Zuckeralkohole, Zucker allgemein oder auch Magnesiumstearat. Das erfindungsgemäße Präparat kann gegebenenfalls auch oberflächenbeschichtet werden.

Die Herstellung der festen, peroral applizierbaren Arzneimittelform, insbesondere Tabletten, kann nach üblichen im Stand der Technik bekannten Verfahren erfolgen. Hierbei ist beispielsweise die Direktpressung der nicht vorbehandelten Hilfs- und Wirkstoffe zu nennen. Darüber hinaus ist selbstverständlich auch die Granulierung einzelner oder mehrerer Rezepturbestandteile vor der Verpreßung möglich.

### Ausführungsbeispiel:

Aus 102,9 mg Tilidin x HCl, 8,8 mg Naloxon x HCl, 16 mg Citronensäure, 199,3 mg einer Hydroxyalkylcellulose (Metocell® K 100 M), 50 mg mikrokristalline Cellulose (Avicel® PH 102) und 4,0 mg Magnesiumstearat wurde durch Direktpressung eine Tablette hergestellt.

Die Freisetzungsraten wurden entsprechend der Beschreibung wie folgt bestimmt:

| Stunden | Tilidin (%) | Naloxon (%) |
|---|---|---|
| 1 | 25 | 20 |
| 2 | 35 | 30 |
| 4 | 60 | 55 |
| 6 | 75 | 65 |
| 8 | 85 | 78 |

## Patentansprüche

1. Feste, peroral applizierbare Schmerzmittel, enthaltend Tilidin als Salz eines Komplexbildners für 2- oder 3-wertige Metallkationen, ausgewählt aus organischen Säuren und einen Morphinantagonisten in einer Cellulosederivatmatrix als Retardiermittel.

2. Schmerzmittel nach Anspruch 1, **dadurch gekennzeichnet, dass** der Komplexbildner ausgewählt ist aus Dicarbonsäuren und/oder Tricarbonsäuren, gegebenenfalls hydroxysubstituiert, beispielsweise Citronensäure und/oder Weinsäure.

3. Feste, peroral applizierbare Schmerzmittel, enthaltend ein pharmakologisch unbedenkliches Tilidinsalz, **zusätzlich** einen Komplexbildner für 2- oder 3-wertige Metallkationen und einen Morphinantagonisten in einer Cellulosederivatmatrix als Retardiermittel.

4. Schmerzmittel nach Anspruch 3, **dadurch gekennzeichnet, dass** sich die Säure des Tilidinsalzes von Orthophosphorsäure, Schwefelsäure und/oder Salzsäure ableitet.

5. Schmerzmittel nach einem der Ansprüche 1 bis 4, enthaltend Komplexbildner in einer Menge von 0,1 bis 40 Gew.-%, bezogen auf die Masse der Gesamtzubereitung.

6. Schmerzmittel nach einem der Ansprüche 1 bis 5, **dadurch gekennzeichnet, dass** der Morphinantagonist Naloxonhydrochlorid ist.

7. Schmerzmittel nach einem der Ansprüche 1 bis 6, **dadurch gekennzeichnet, dass** das Retardiermittel ausgewählt ist aus Alkylcellulosen und Hydroxyalkylcellulosen mit 1 bis 6 C-Atomen im Alkyl- oder Hydroxyalkylrest.

8. Schmerzmittel nach Anspruch 7, **dadurch gekennzeichnet, dass** die Alkylcellulosen ausgewählt sind aus Methylcellulose und/oder Ethylcellulose.

9. Schmerzmittel nach Anspruch 7, **dadurch gekennzeichnet, dass** die Hydroxyalkylcellulosen ausgewählt sind aus Hydroxyethylcellulose und/oder Hydroxypropylcellulose.

10. Schmerzmittel nach einem der Ansprüche 1 bis 9, **dadurch gekennzeichnet, dass** die Freisetzungsgeschwindigkeiten des Tilidins nach
1 Stunde 30 Gew.- %,
2 Stunden 25 bis 40 Gew.-%,
4 Stunden 50 bis 70 Gew.-%,
6 Stunden 60 bis 80 Gew.-% und
8 Stunden 70 bis 90 Gew.-%
beträgt.

11. Schmerzmittel nach einem der Ansprüche 1 bis 10, **dadurch gekennzeichnet, dass** die in-vitro Freisetzungsrate des Tilidins und des Morphinantagonisten im wesentlichen gleich sind.

12. Schmerzmittel nach einem der Ansprüche 1 bis 10, **dadurch gekennzeichnet, dass** sich die Freisetzungsraten von Tilidin- und Morphinantagonist nach
1 Stunde um höchstens 25 %, bezogen auf Tilidin,
2 Stunden um höchstens 20 %, bezogen auf Tilidin,
4 Stunden um höchstens 15 %, bezogen auf Tilidin,
6 Stunden um höchstens 12 %, bezogen auf Tilidin und
8 Stunden um höchstens 10 %, bezogen auf Tilidin
unterscheiden.

## Claims

1. Solid perorally applicable analgesics containing tilidine as a salt of a complexing agent for di- or trivalent metal cations selected from organic acids and a morphine antagonist in a cellulose derivative matrix as a retarding means.

2. The analgesics according to claim 1, **characterized in that** said complexing agent is selected from dicarboxylic acids and/or tricarboxylic acids, optionally hydroxy-substituted, for example, citric acid and/or tartaric acid.

3. Solid perorally applicable analgesics containing a pharmacologically acceptable tilidine salt, additionally a complexing agent for di- or trivalent metal cations, and a morphine antagonist in a cellulose derivative matrix as a retarding means.

4. The analgesics according to claim 3, **characterized in that** the acid of said tilidine salt is derived from ortho-phosphoric acid, sulfuric acid and/or hydrochloric acid.

5. The analgesics according to any of claims 1 to 4, containing complexing agents in an amount of from 0.1 to 40% by weight, based on the weight of the total formulation.

6. The analgesics according to any of claims 1 to 5, **characterized in that** said morphine antagonist is naloxone hydrochloride.

7. The analgesics according to any of claims 1 to 6, **characterized in that** said retarding means is selected from alkylcelluloses and hydroxyalkylcelluloses having from 1 to 6 carbon atoms in the alkyl or hydroxyalkyl residue.

8. The analgesics according to claim 7, **characterized in that** said alkylcelluloses are selected from methylcellulose and/or ethylcellulose.

9. The analgesics according to claim 7, **characterized in that** said hydroxyalkylcelluloses are selected from hydroxyethylcellulose and/or hydroxypropylcellulose.

10. The analgesics according to any of claims 1 to 9, **characterized in that** the release rates of the tilidine are:
30% by weight after 1 hour;
from 25 to 40% by weight after 2 hours;
from 50 to 70% by weight after 4 hours;
from 60 to 80% by weight after 6 hours; and
from 70 to 90% by weight after 8 hours.

11. The analgesics according to any of claims 1 to 10, **characterized in that** the in-vitro release rates of tilidine and of the morphine antagonist are essentially the same.

12. The analgesics according to any of claims 1 to 10, **characterized in that** the release rates of tilidine and of the morphine antagonist are different:
by at most 25%, based on tilidine, after 1 hour;
by at most 20%, based on tilidine, after 2 hours;
by at most 15%, based on tilidine, after 4 hours;
by at most 12%, based on tilidine, after 6 hours; and
by at most 10%, based on tilidine, after 8 hours.

## Revendications

1. Analgésiques solides à application orale, contenant la tilidine en forme de sel d'un agent complexant pour des cations de métaux divalents ou trivalents, choisi parmi les acides organiques et un antagoniste morphinique dans une matrice de dérivé cellulosique comme moyen de retardation.

2. Analgésiques selon la revendication 1, **caractérisés en ce que** ledit agent complexant est choisi parmi les acides dicarboxyliques et/ou tricarboxyliques, éventuellement substitués par hydroxy, par exemple, l'acide citrique et/ou l'acide tartrique.

3. Analgésiques solides à application orale, contenant un sel de tilidine pharmacologiquement acceptable, en plus un agent complexant pour des cations de métaux divalents ou trivalents, et un antagoniste morphinique dans une matrice de dérivé cellulosique comme moyen de retardation.

4. Analgésiques selon la revendication 3, **caractérisés en ce que** l'acide dudit sel de tilidine est dérivé de l'acide ortho-phosphorique, de l'acide sulfurique et/ou de l'acide chlorhydrique.

5. Analgésiques selon l'une quelconque des revendications 1 à 4, contenant des agents complexants dans une quantité de 0,1 à 40% en poids, par rapport au poids de la formulation totale.

6. Analgésiques selon l'une quelconque des revendications 1 à 5, **caractérisés en ce que** ledit antagoniste morphinique est le chlorhydrate de naloxone.

7. Analgésiques selon l'une quelconque des revendications 1 à 6, **caractérisés en ce que** ledit moyen de retardation est choisi parmi les alkylcelluloses et hydroxyalkylcelluloses comprenant de 1 à 6 atomes de carbone dans leur résidu alkyl ou hydroxyalkyl.

8. Analgésiques selon la revendication 7, **caractérisés en ce que** lesdits alkylcelluloses sont choisis parmi la méthylcellulose et/ou l'éthylcellulose.

9. Analgésiques selon la revendication 7, **caractérisés en ce que** lesdits hydroxyalkylcelluloses sont choisis parmi l'hydroxyéthylcellulose et/ou l'hydroxypropylcellulose.

10. Analgésiques selon l'une quelconque des revendications 1 à 9, **caractérisés en ce que** les vitesses de libération du tilidine sont:
30% en poids après 1 heure;
de 25 à 40% en poids après 2 heures;
de 50 à 70% en poids après 4 heures;
de 60 à 80% en poids après 6 heures; et
de 70 à 90% en poids après 8 heures.

11. Analgésiques selon l'une quelconque des revendications 1 à 10, **caractérisés en ce que** les vitesses de libération in-vitro du tilidine et dudit antagoniste morphinique sont essentiellement égales.

12. Analgésiques selon l'une quelconque des revendications 1 à 10, **caractérisés en ce que** les vitesses de libération du tilidine et dudit antagoniste morphinique sont différentes:
de 25% au plus, par rapport à la tilidine, après 1 heure;
de 20% au plus, par rapport à la tilidine, après 2 heures;
de 15% au plus, par rapport à la tilidine, après 4 heures;
de 12% au plus, par rapport à la tilidine, après 6 heures; et
de 10% au plus, par rapport à la tilidine, après 8 heures.
